(19) **Europäisches Patentamt**
**European Patent Office**
**Office européen des brevets**

(11) Veröffentlichungsnummer: **0 325 925 B1**

(12) # EUROPÄISCHE PATENTSCHRIFT

(45) Veröffentlichungstag der Patentschrift:
21.08.91 Patentblatt 91/34

(51) Int. Cl.$^5$: **C07C 51/43, C07C 65/11**

(21) Anmeldenummer: **89100236.2**

(22) Anmeldetag: **07.01.89**

(54) **Verfahren zur Reinigung von 2-Hydroxy-naphthalin-6-carbon-säure.**

(30) Priorität: **15.01.88 DE 3800989**

(43) Veröffentlichungstag der Anmeldung:
**02.08.89 Patentblatt 89/31**

(45) Bekanntmachung des Hinweises auf die
Patenterteilung:
**21.08.91 Patentblatt 91/34**

(84) Benannte Vertragsstaaten:
**BE CH DE FR GB IT LI NL**

(56) Entgegenhaltungen:
**DE-A- 2 332 064
DE-C- 436 524
FR-A- 2 466 450
US-A- 2 132 356
BEILSTEINS HANDBUCH DER ORGANI-
SCHEN CHEMIE, 4. Auflage, Band 10, 4.Ergän-
zungswerk, 2. Teil**

(73) Patentinhaber : **HOECHST
AKTIENGESELLSCHAFT
Postfach 80 03 20
W-6230 Frankfurt am Main 80 (DE)**

(72) Erfinder : **von Plessen, Helmold, Dr.
Kugelhermstrasse 16
W-6240 Königstein/Taunus (DE)**
Erfinder : **Rittner, Siegbert, Dr.
Kornblumenweg 5
W-6082 Mörfelden-Walldorf (DE)**
Erfinder : **Volk, Heinrich, Dr.
Am weissen Stein 1
W-6368 Bad Vilbel (DE)**
Erfinder : **Wykypiel, Werner, Dr.
Egerstrasse 7
W-6054 Rodgau (DE)**
Erfinder : **Neeb, Rudolf, Dr.
Am Klingenrain 23
W-6050 Offenbach (DE)**

## Beschreibung

Die Erfindung betrifft ein Verfahren zur Reinigung von 2-Hydroxy-naphthalin-6-carbonsäure (im folgenden auch kurz als "2,6-Säure" bezeichnet) durch Umkristallisation.

Die für die Herstellung von speziellen temperaturbeständigen Kunststoffen als Monomeres verwendete 2,6-Säure muß für die Polykondensation "fibre grade quality" besitzen, d.h. sie muß von hoher Reinheit sein. Diese Forderung ist jedoch schwierig zu erfüllen, da das Rohprodukt nach einer Variante der Kolbe-Schmitt-Reaktion durch Umsetzung des Kaliumsalzes von 2-Naphthol mit $CO_2$ gewonnen wird (US-PS 1593816, US-PS 4287357, EP-PS 0081753). Bei dieser Reaktion entstehen beträchtliche Anteile an Zersetzungsprodukten (Teere und Harze), die sich ebenso wie die entstehenden Nebenprodukte nur schwierig abtrennen lassen.

Bisher wird die Rohsäure nach Abtrennung von 2-Naphthol in aufwendiger Weise aufgearbeitet, und zwar durch Extraktion, Kohleadsorption und Umfällung aus Wasser. Wie weit die hierbei erreichten Qualitäten zu wünschen übrig lassen, zeigen die Handelsprodukte, die statt farblos mehr oder weniger bräunlich sind.

Es wurde nun überraschenderweise gefunden, daß 2,6-Säure mit der gewünschten hohen Reinheit gewonnen werden kann, wenn man die Rohsäure aus bestimmten, mit Wasser mischbaren Ethern oder deren wäßrigen Lösungen umkristallisiert.

Gegenstand der Erfindung ist somit ein Verfahren zur Reinigung von 2-Hydroxy-naphthalin-6-carbonsäure durch Umkristallisation, das dadurch gekennzeichnet ist, daß man die rohe Säure aus mit Wasser mischbaren linearen oder cyclischen aliphatischen Ethern, aliphatischen Polyethern oder aliphatischen Hydroxy-ethern oder aus mindestens 10 gew.-%igen wäßrigen Lösungen dieser Ether umkristallisiert.

Besonders vorteilhaft ist es, daß durch die Behandlung roher 2,6-Säure mit den Ethern oder ihren wäßrigen Lösungen außer den färbenden Bestandteilen auch kritische Verunreinigungen der 2,6-Säure wie 2-Naphthalinsulfonsäure, 2-Naphthol, 2-Hydroxy-naphthalin-3-carbonsäure, 6-Hydroxy-naphthalin-2,7-dicarbonsäure, 2,2'-Dihydroxy-1,1'-dinaphthyl abgetrennt werden.

Eine Methode des erfindungsgemäßen Kristallisationsverfahrens besteht darin, die rohe 2,6-Säure in den genannten Ethern oder deren wäßrigen Lösungen in der Hitze aufzulösen und dann durch Abkühlung die gereinigte Säure auskristallisieren zu lassen. Nach einer anderen Methode werden Lösungen der Rohsäure in den genannten Ethern mit soviel Wasser versetzt, daß Kristallisation eintritt. Weiter kann man aus Lösungen der Rohsäure in den genannten Ethern oder ihren wäßrigen Lösungen soviel Ether abdestillieren, daß Kristallisation eintritt.

Vor der erfindungsgemäßen Kristallisation wird vorzugsweise die rohe 2,6-Säure von der Hauptmenge des etwa vorhandenen 2-Naphthols befreit. Man kann beispielsweise bei der Umsetzung des Kaliumsalzes von 2-Naphthol mit $CO_2$ das als Nebenprodukt entstandene 2-Naphthol bei vermindertem Druck (z.B. 50-65 mbar) und erhöhter Temperatur (z.B. 260°C) aus der Carboxylierungsschmelze abdestillieren.

Die Kristallisation von 2,6-Säure kann sowohl bei Raumtemperatur als auch bei erhöhten (bis zum Siedepunkt) oder verminderten Temperaturen (bis etwa – 20°C) erfolgen. Die Kristallisation ist auch bei erhöhtem Druck möglich, unter entsprechender Erhöhung des Siedepunktes.

Das erfindungsgemäße Kristallisationsverfahren kann sowohl diskontinuierlich als auch kontinuierlich ausgeübt werden. Dabei kann entweder die Kühlungs- oder Verdampfungskristallisation oder die sogenannte Vakuumkristallisation angewandt werden. Hierbei kann es von Vorteil sein, unter Inertgasatmosphäre zu arbeiten. Als Inertgas kommt dabei vor allem Stickstoff in Frage. Daneben können für diesen Zweck Kohlendioxid oder Argon eingesetzt werden.

Für das erfindungsgemäße Verfahren können beispielsweise folgende Ether oder ihre Gemische eingesetzt werden, und zwar mit oder ohne Zusatz von Wasser :

— Monoethylenglykoldimethylether (1,2-Dimethoxyethan)
— Diethylenglykoldimethylether (2,2'-Dimethoxy-diethylether)
— Triethylenglykoldimethylether (1,2-Bis-(2'-methoxyethoxy)-ethan)
— Tetraethylenglykoldimethylether (Homologengemisch)
— Diethylenglykol
— Triethylenglykol
— Polyethylenglykol, z.B. Polyethylenglykol 400 (Molmasse 380-420)
— Pentaethylenglykoldimethylether
— Triethylenglykol-methyl-ethyl-ether
— Tetraethylenglykol-methyl-tert.-butylether
— Monoethylenglykolmonomethylether
— Diethylenglykolmonomethylether
— Triethylenglykolmonomethylether

— Tetraethylenglykolmonomethylether

— 1.4-Dioxan

— 1.3.5-Trioxan

— Trioxepan

— Tetroxan

— Bicyclotetroxan

— Dimethylbicyclotetroxan

— Methyldicyclotetroxan

— wasserlösliche kurzkettige Ethylenoxid -Propylenoxid-Copolymere

— 2-Methoxy-butanol-(1)

— Glycerin-monomethylether

— Glycerin-dimethylether

— Glycerin-trimethylether.

Vorzugsweise verwendet man 1,4-Dioxan oder eine mindestens 10 gew.-%ige wäßrige Lösung eines der folgenden Ether : 1,4-Dioxan, Diethylenglykol, Triethylenglykol, Polyethylenglykol oder Ethylenglykoldimethylether. Besonders geeignet sind mindestens 10 gew.-%ige wäßrige Lösungen von 1,4-Dioxan oder Ethylenglykoldimethylether, vor allem aber die erstgenannte Lösung. Der 1,4-Dioxan-Gehalt dieser Lösung sollte i.allg. 10-90 Gew.-%, vorzugsweise 20-90 Gew.-%, insbesondere 35-90 Gew.-% betragen. Lösungen mit einem 1,4-Dioxan-Gehalt über 90 Gew.-% oder wasserfreies 1,4-Dioxan sind zwar technisch genauso gut geeignet, aber ökonomisch ungünstiger wegen des höheren Ethereinsatzes bei gleichem technischen Effekt. Für die wäßrigen Lösungen des Ethylenglykoldimethylethers und der anderen genannten Ether gilt dasselbe wie für die wäßrigen Lösungen des 1,4-Dioxans, was die Ether-Gehalte der Lösungen (i.allg. 10-90, vorzugsweise 20-90, insbesondere 35-90 Gew.-%) und die Eignung der reinen Ether betrifft.

Der große Vorteil des erfindungsgemäßen Verfahrens liegt nicht nur in der hohen Reinheit der gewonnenen 2,6-Säure, sondern auch darin, daß die Reinigung aus nur einer verfahrenstechnischen Grundoperation besteht.

Die gewonnene hochreine 2,6-Säure stellt ein wertvolles Produkt dar. Aus ihr und p-Hydroxybenzoesäure können beispielsweise aromatische Polyester hergestellt werden, die sich zu temperaturbeständigen Kunststoffen oder Fasern verarbeiten lassen (US-PS 4393191). Außer als Monomeres für Kunststoffe, Fasern und Fäden kann die 2,6-Säure auch als Synthesebaustein, z.B. für Farbstoffe oder Textilhilfsmittel dienen.

Das erfindungsgemäße Verfahren wird anhand der folgenden Beispiele erläutert. Die Farbzahl der rohen bzw. gereinigten 2,6-Säure wurde dabei wie folgt bestimmt :

1 g 2,6-Säure wurde in 80 ml 100%iger Essigsäure im Ultraschallbad gelöst und die Lösung mit 100%iger Essigsäure auf 100 ml aufgefüllt. Anschließend wurde mit einem Spektralphotometer die Absorption bei 400 nm bestimmt. Die Messung erfolgte stets innerhalb von 2 Stunden nach Beginn der Auflösung der Probe. Die Farbzahl bei 400 nm ergab sich dann gemäß der Formel

$$\text{Farbzahl (400 nm)} = \frac{\text{Absorption bei 400 nm}}{(1\ \text{cm}) \cdot \text{Konzentration der Probe in ppm}}$$

wobei 1 g auf 100 ml als 10000 ppm gerechnet wird. In den Beispielen 1-10 hatte die rohe 2,6-Säure stets die Farbzahl $25{,}6 \cdot 10^{-6}$, in Beispiel 11 die Farbzahl $61{,}2 \cdot 10^{-6}$.

**Beispiel 1**

12,5 g rohe 2,6-Säure wurden mit 100 ml Wasser verrührt und unter Zugabe von 40 ml Ethylenglykoldimethylether und Erwärmen gelöst. Nach Behandlung der Lösung mit 2,5 g Aktivkohle und Filtration schieden sich beim Abkühlen Kristalle aus, die abfiltriert wurden. Die gewonnene Substanz wurde dann aus 150 ml 35 vol-%igem Ethanol umkristallisiert. Nach Trocknung wurden 8,0 g kristalline 2,6-Säure erhalten : Schmelzpunkt 246-247,5°C, Farbzahl $5{,}3 \cdot 10^{-6}$.

## Beispiel 2

10 g rohe 2,6-Säure wurden in einem Gemisch aus 100 ml Wasser und 45 ml Diethylenglykol unter Erwärmen gelöst. Die Lösung wurde 30 Minuten lang unter Rückfluß mit 0,16 g Aktivkohle gekocht und anschließend filtriert. Das aus der Lösung auskristallisierte Produkt wurde abfiltriert und mit Wasser gewaschen. Nach Trocknung ergab sich eine Farbzahl von 10,7 · $10^{-6}$.

## Beispiel 3

25 g rohe 2,6-Säure wurden mit 200 ml Wasser unter Rühren erwärmt und durch Zugabe von 150 ml 1,4-Dioxan gelöst. Nach Behandlung der Lösung mit 5 g Aktivkohle wurde abgekühlt und die auskristallisierte 2,6-Säure gewonnen und mit einem Gemisch aus 100 Volumenteilen Wasser und 60 Volumenteilen 1,4-Dioxan gewaschen. Das Kristallisat wurde aus 200 ml 35 vol-%igem Ethanol umkristallisiert und gewaschen.

Nach Trocknung betrug die Ausbeute 18 g farblose Kristalle. Der Schmelzpunkt war 247-248,5°C, die Farbzahl 1,4 · $10^{-6}$. Verunreinigungen (in Gew.-%) : 2-Naphthalinsulfonsäure unter 0,002% ; 2-Hydroxy-naphthalin-3-carbonsäure unter 0,05% ; 2-Naphthol unter 0,05% ; 6-Hydroxy-naphthalin-2,7-dicarbonsäure 0,05% ; 2,2'-Dihydroxy-1,1'-dinaphthyl unter 0,05%.

Die rohe 2,6-Säure enthielt folgende Verunreinigungen (in Gew.-%) : 2-Naphthalinsulfonsäure unter 0,002% ; 2-Hydroxy-naphthalin-3-carbonsäure 0,227% ; 2-Naphthol 0,065% ; 6-Hydroxy-naphthalin-2,7-dicarbonsäure 0,421% ; 2,2'-Dihydroxy-1,1'-dinaphthyl 0,018%.

## Beispiel 4

12,5 g rohe 2,6-Säure wurden in 100 ml Wasser und 55 ml Diethylenglykol unter Erwärmen gelöst. Nach 13-minütiger Behandlung der heißen Lösung mit 1 g Aktivkohle wurde filtriert. Im Filtrat ausgeschiedene Kristalle wurden durch Erwärmen wieder in Lösung gebracht. Anschließend ließ man langsam auskristallisieren. Nach Filtration wurde mit 25 vol-%igem Ethanol gewaschen. Ausbeute nach Trocknung : 9,7 g.

Das aus 120 ml 35 vol-%igem Ethanol umkristallisierte Produkt zeigte eine Farbzahl von 9,2 · $10^{-6}$.

## Beispiel 5

12,5 g rohe 2,6-Säure wurden in 100 ml Wasser und 75 ml 1,4-Dioxan unter Erwärmen gelöst. Nach Behandlung der Lösung mit 2,5 g Aktivkohle und Filtration kristallisierten aus der Lösung 11,9 g 2,6-Säure in Form eines Addukts mit Dioxan aus, die eine Farbzahl von 3,4 · $10^{-6}$ aufweisen.

## Beispiel 6

12,5 g rohe 2,6-Säure wurden in 100 ml Wasser sowie 40 ml Ethylenglykoldimethylether unter Erwärmen gelöst und mit 2,5 g Aktivkohle behandelt. Die filtrierte Lösung wurde dann zur Kristallisation abgekühlt. Das mit etwas verdünntem Ethanol gewaschene Kristallisat wurde dann aus einem wie oben zusammengesetzten Gemisch von Wasser und Ethylenglykoldimethylether nochmal umkristallisiert. Ausbeute : 8,1 g, Farbzahl 8,5 · $10^{-6}$.

## Beispiel 7

12,5 g rohe 2,6-Säure wurden in 100 ml Wasser und 50 ml 1,3-Dioxolan unter Erwärmen gelöst. Nach Behandlung der Lösung mit 2,5 g Aktivkohle wurde filtriert, die Lösung abgekühlt und das Kristallisat gewonnen. Nach Umkristallisieren aus 120 ml 35 vol.-%igem Ethanol wurden 7,3 g Ausbeute erhalten. Schmelzpunkt : 246-247.9°C, Farbzahl : 7,8 · $10^{-6}$.

## Beispiel 8

12,5 g rohe 2,6-Säure wurden in 100 ml Wasser sowie 50 ml 1,3-Dioxolan unter Erwärmen gelöst und mit 2,5 g Aktivkohle behandelt. Aus der abgekühlten Lösung wurde das Kristallisat abfiltriert, mit 35 vol.-%igem Ethanol gewaschen und erneut in 100 ml Wasser sowie 50 ml 1,3-Dioxolan gelöst und daraus umkristallisiert. Ausbeute : 5,1 g, Farbzahl : 6,8 · $10^{-6}$.

4

**Beispiel 9**

12,5 g rohe 2,6-Säure wurden unter Erärmen in 180 ml 1,4-Dioxan gelöst. Nach Behandlung mit 2,5 g Aktivkohle, Filtration und Abkühlung der Lösung wurden daraus 9,1 g kristallisierte 2,6-Säure gewonnen. Umkristallisation aus 100 ml 35 vol.-%igem Ethanol und Waschen mit demselben Lösemittel ergab nach Trocknung 6,7 g Ausbeute. Die Farbzahl war $3,3 \cdot 10^{-6}$.

**Beispiel 10**

25 g rohe 2,6-Säure wurden in 96 ml Dioxan und 24 ml Wasser unter Rühren heiß gelöst. Die mit 1 g Aktivkohle behandelte und filtrierte Lösung ergab nach Abkühlung kristallisierte 2,6-Säure, die abfiltriert und mit 25 vol.-%igem Ethanol gewaschen wurde. Nach Trocknung betrug die Ausbeute 18,2 g und die Farbzahl $3,6 \cdot 10^{-6}$.

**Beispiel 11**

25 g rohe 2,6-Säure (Farbzahl $61,2 \cdot 10^{-6}$) wurden in 200 ml Wasser und 150 ml Dioxan unter Rühren und Erwärmen gelöst. Nach Behandlung der Lösung mit 2 g Aktivkohle und Filtration ergab Abkühlung ein Kristallisat, das abgetrennt und mit 25 vol.-%igem Ethanol gewaschen wurde. Das Produkt wurde aus 180 ml Wasser und 135 ml Dioxan unter Behandlung mit 1 g Aktivkohle erneut umkristallisiert. Nach Waschen mit 25 vol.-%igem Ethanol und Trocknen wurden 21,5 g gereinigte 2,6-Säure mit einer Farbzahl von $2,7 \cdot 10^{-6}$ erhalten.

**Patentansprüche**

1. Verfahren zur Reinigung von 2-Hydroxy-naphthalin-6-carbonsäure durch Umkristallisation, dadurch gekennzeichnet, daß man die rohe Säure aus mit Wasser mischbaren linearen oder cyclischen aliphatischen Ethern, aliphatischen Polyethern oder aliphatischen Hydroxyethern oder gemischen davon oder aus mindestens 10 gew.-%igen wäßrigen Lösungen dieser Ether umkristallisiert.

2. Verfahren nach Anspruch 1, dadurch gekennzeichnet, daß man aus einer 20-90 gew.-%igen wäßrigen Lösung von Ethylenglykoldimethylether umkristallisiert.

3. Verfahren nach Anspruch 1, dadurch gekennzeichnet, daß man aus einer 20-90 gew.-%igen wäßrigen Lösung von 1,4-Dioxan umkristallisiert.

4. Verfahren nach Anspruch 1, dadurch gekennzeichnet, daß man aus einer 20-90 gew.-%igen wäßrigen Lösung von Diethylenglykol umkristallisiert.

5. Verfahren nach Anspruch 1, dadurch gekennzeichnet, daß man aus einer 20-90 gew.-%igen wäßrigen Lösung von Triethylenglykol umkristallisiert.

6. Verfahren nach Anspruch 1, dadurch gekennzeichnet, daß man aus einer 20-90 gew.-%igen wäßrigen Lösung von Polyethylenglykol umkristallisiert.

7. Verfahren nach Anspruch 1, dadurch gekennzeichnet, daß man aus einer 35-90 gew.-%igen wäßrigen Lösung von Ethylenglykoldimethylether umkristallisiert.

8. Verfahren nach Anspruch 1, dadurch gekennzeichnet, daß man aus einer 35-90 gew.%igen wäßrigen Lösung von 1,4-Dioxan umkristallisiert.

9. Verfahren nach Anspruch 1, dadurch gekennzeichnet, daß man aus einer mindestens 90 gew.-%igen wäßrigen Lösung von 1,4-Dioxan oder aus wasserfreiem 1,4-Dioxan umkristallisiert.

**Claims**

1. A process for the purification of 2-hydroxy-naphthalene-6-carboxylic acid by recrystallization, which comprises recrystallizing the crude acid from watermiscible linear or cyclic aliphatic ethers, aliphatic polyethers or aliphatic hydroxy ethers or mixtures thereof or from at least 10% strength by weight aqueous solutions of these ethers.

2. The process as claimed in claim 1, wherein the recrystallization is carried out from a 20-90% strength by weigth aqueous solution of ethylene glycol dimethyl ether.

3. The process as claimed in claim 1, wherein the recrystallization is carried out from a 20-90% strength by weight aqueous solution of 1,4-dioxane.

4. The process as claimed in claim 1, wherein the recrystallization is carried out from a 20-90% strength by weight aqueous solution of diethylene glycol.

5. The process as claimed in claim 1, wherein the recrystallization is carried out from a 20-90% strength by weight aqueous solution of triethylene glycol.

6. The process as claimed in claim 1, wherein the recrystallization is carried out from a 20-90% strength by weight aqueous solution of polyethylene glycol.

7. The process as claimed in claim 1, wherein the recrystallization is carried out from a 35-90% strength by weight aqueous solution of ethylene glycol dimethyl ether.

8. The process as claimed in claim 1, wherein the recrystallization is carried out from a 35-90% strength by weight aqueous solution of 1,4-dioxane.

9. The process as claimed in claim 1, wherein the recrystallization is carried out from an at least 90% strength by weight aqueous solution of 1,4-dioxane or from anhydrous 1,4 -dioxane.

## Revendications

1. Procédé pour purifier l'acide hydroxy-2 naphtalène-carboxylique-6 par recristallisation, procédé caractérisé en ce qu'on recristallise l'acide brut dans des éthers aliphatiques linéaires ou cycliques, des polyéthers aliphatiques ou des hydroxy-éthers aliphatiques, miscibles à l'eau, ou dans des mélanges de tels éthers, ou encore dans des solutions aqueuses à au moins 10% en poids de ces éthers.

2. Procédé selon la revendication 1 caractérisé en ce qu'on recristallise dans une solution aqueuse à 20-90% en poids d'éther diméthylique de l'éthylène-glycol.

3. Procédé selon la revendication 1 caractérisé en ce qu'on recristallise dans une solution aqueuse à 20-90% en poids de dioxanne-1,4.

4. Procédé selon la revendication 1 caractérisé en ce qu'on recristallise dans une solution aqueuse à 20-90% en poids de diéthylène-glycol.

5. Procédé selon la revendication 1 caractérisé en ce qu'on recristallise dans une solution aqueuse à 20-90% en poids de triéthylène-glycol.

6. Procédé selon la revendication 1 caractérisé en ce qu'on recristallise dans une solution aqueuse à 20-90% en poids d'un poly-éthylène-glycol.

7. Procédé selon la revendication 1 caractérisé en ce qu'on recristallise dans une solution aqueuse à 35-90% en poids d'éther diméthylique de l'éthylène-glycol.

8. Procédé selon la revendication 1 caractérisé en ce qu'on recristallise dans une solution aqueuse à 35-90% en poids de dioxanne-1,4.

9. Procédé selon la revendication 1 caractérisé en ce qu'on recristallise dans une solution aqueuse à au moins 90% en poids de dioxanne-1,4 ou dans du dioxanne-1,4 anhydre.